# EUROPEAN PATENT APPLICATION

(11) **EP 4 825 390 A1**
(43) Date of publication of application: **21.10.2026**
(21) Application number: 25171211.3
(22) Date of filing: 17.04.2025
(51) Int. Cl.: A61K 38/16, A61K 39/02, C07K 14/315

(54) **METHOD FOR PRODUCING A RECOMBINANT FUSION PROTEIN**

(71) Applicant: MinervaX ApS, 2000 Frederiksberg (DK)
(72) Inventor: BOMHOLT, Julie, 2920 CHARLOTTENLUND (DK); KANTSØ, Bjørn, 4300 HOLBÆK (DK)
(74) Representative: Öberg & Sjöberg AB

(57) **Abstract**

The technology proposed herein is directed to a fermentation method for producing a recombinant fusion protein comprising a first amino acid sequence of the N-terminal region of a first group B *Streptococcus* surface protein, linked to a second amino acid sequence of the N-terminal region of a second group B *Streptococcus* surface protein. The fermentation method comprises growing a host cell comprising a gene encoding the fusion protein under control of the *lac* repressor and inducing expression of the recombinant fusion protein when the acceptance criterion for induction is reached, harvesting, concentrating and washing the cells before they are disrupted and a cell homogenate comprising the recombinant fusion protein is obtained. The technology proposed herein is also directed to a recombinant fusion protein obtained or obtainable by the fermentation method.

## Description

### TECHNICAL FIELD

The technology proposed herein relates to the fields of microbiology and vaccine technology and concerns the production of a recombinant fusion protein capable of conferring immunity to group B Streptococcus infections. More particularly, the technology proposed herein relates to the production of such recombinant fusion proteins and the recombinant fusion proteins produced by specific production methods as such.

### BACKGROUND

Group B Streptococcus (*Streptococcus agalactiae)* (GBS) is the major cause of invasive bacterial infections, including meningitis, in the neonatal period. In the United States alone, there are now about 5000 cases per year of invasive disease caused by this bacterium. These infections have an overall mortality of about 10%, and many of the infants that survive have permanent neurological sequelae. In view of this, a large effort has been made to find methods of prevention and treatment and to analyze the mechanisms by which GBS cause infections.

GBS can also cause mastitis in cows, a bovine disease that is of considerable economical importance. Development of a vaccine against GBS infections is therefore of interest also in veterinary medicine.

About 20 % of all women are vaginal carriers of GBS, and vertical transmission from the maternal genital tract is probably the most common source of infection in neonatal disease caused by this bacterium. However, only about 1 % of the infants that are colonized by the GBS at birth are afflicted by serious infection. Other factors than exposure to the bacterium during birth must therefore contribute to the development of neonatal disease.

Group B streptococcal strains are divided into nine serotypes (la, lb, and II-VIII) based on the structure of the polysaccharide capsule. The four "classical" serotypes la, Ib, II, and III occur in roughly equal proportions among strains in the normal flora, but type III is the clinically most important serotype, in particular because it causes most cases of meningitis. Because the capsule is a known virulence factor, it has been studied in considerable detail, in particular in type III strains. Efforts have been made to develop a vaccine, in which the type III polysaccharide capsule would be an essential component.

The document Lindahl et al, Nonimmunodominant Regions Are Effective As Building Blocks In A Streptococcal Fusion Protein Vaccine, Cell Host & Microbe 2, 427-434, December 2007, discloses a fusion protein comprising N-terminal regions of the group B Streptococcus surface proteins Rib and AlpC.

Similarly, WO 2008127179 describes a fusion protein comprising at least one first N-terminal region fragment of a group B Streptococcus surface protein or analogue, homologue, derivative or immunologically related amino acid sequence or fragments thereof, which is fused to at least one second N-terminal region fragment of a group B Streptococcus surface protein or analogue, homologue, derivative or immunologically related amino acid sequence or fragments thereof, wherein the first and second at least one N-terminal region fragments of group B Streptococcus surface proteins derive from different group B Streptococcus strains, and wherein the fusion protein is capable of eliciting protective immunity against group B Streptococcus.

Despite the advances in the progress towards a vaccine suitable for prevention of GBS disease, there is still a need for further methods and vaccines for prevention and treatment of GBS infections. Also, a successful vaccine strategy relies on the ability to efficiently being able to produce sufficient amounts of vaccine of good quality.

Accordingly, it is a primary objective of the technology proposed herein to provide an improved method for the production of recombinant fusion proteins, in particular recombinant fusion proteins that are to be used for immunisation.

Another objective of the technology proposed herein is to provide recombinant fusion proteins that are suitable to use for immunization.

The means of accomplishing each of the above objectives as well as others will become apparent from the description which follows hereafter.

### SUMMARY

A first aspect of the technology proposed herein concerns a fermentation method for producing a recombinant fusion protein comprising N-terminal regions of group B Streptococcus surface proteins. The method comprises the steps of:
i) growing a host cell, such as an *E. coli* host cell, said host cell comprising a gene encoding said recombinant fusion protein under control of the Lacl, in a growth medium;
ii) when the cell density reaches an OD₆₀₀ of from about 3.5 to about 6, such as from about 4 to about 5.5, such as from about 4 to about 5, such from about 4.3 to about 4.8, such as about 4.5, adding IPTG to induce expression of said gene encoding said recombinant fusion protein;
iii) harvesting, concentrating and washing and the grown cells by tangential flow filtration;
iv) disrupting the cells to prepare a homogenate comprising said recombinant fusion protein.

The technology proposed herein is based on the discovery that conventional large-scale methods for fermentation, including cell harvesting and preparation of cell homogenates, did not result in a satisfactory protein product when using methods conventionally used for these purposes for the production of recombinant fusion proteins as defined herein. It was inter alia found that such methods in some cases produced recombinant fusion proteins that were not of sufficiently high quality to be used for immunization. This resulted in recombinant fusion proteins that, although generally having the desired immunogenicity, posed a problem to quality control as they prevented simple quality control assays, e.g. size assays, that could otherwise be used to ascertain whether each batch of produced recombinant fusion proteins had the desired structure.

For example, if host cells allowing for glycosylation of the recombinant fusion proteins were used, the. glycosylation on the produced recombinant fusion proteins caused the produced recombinant fusion proteins to have a range of molecular masses. This range meant that simple size-based assays, e.g. size exclusion chromatography, could not be used to discern a recombinant protein having the correct amino acid sequence structure but being glycosylated from a recombinant protein having an incomplete amino acid sequence but being even more heavily glycosylated since the molecular weight of these proteins would be similar. Additionally, a widely varying extent of glycosylation can also affect the charge of the recombinant fusion protein in different buffers as well as the hydrophobicity of the recombinant protein, thus also potentially causing different results in assays such as electrophoresis and hydrophobic interaction chromatography.

However, by the fermentation method according to the technology proposed herein, it was possible to produce high-quality recombinant fusion proteins, i.e. recombinant fusion proteins having a very narrow spread in properties. The recombinant fusion proteins had a sufficiently high quality to be able to be used for immunization.

In a second aspect, the technology proposed herein is directed to a recombinant fusion protein as defined herein, wherein said recombinant fusion protein has been obtained or is obtainable by a method according to the first aspect of the technology proposed herein.

The technology proposed herein is in a third aspect directed to a fermentation method for producing a recombinant fusion protein with less modifications, wherein said fermentation method comprises the steps of the fermentation method of the first aspect of the technology proposed herein.

The technology proposed herein is in a fourth aspect directed to fermentation method for reducing the number of modifications of a recombinant fusion protein, said fermentation method comprising the steps of the fermentation method of the first aspect of the technology proposed herein.

### BRIEF DESCRIPTION OF THE FIGURES

Figure 1 shows an illustration of the fermentation method of the technology proposed herein.
Figure 2 shows a map over the plasmid pAMJ3074-pAMJ3080, expressing the recombinant fusion protein GBS-NN (NN-GBS in map).
Figure 3 shows a map over the plasmid pUP1712-pUP1716, expressing the recombinant fusion protein GBS-NN2 (Alp1-Alp2-NN-v2 in map).
Figure 4 shows SDS-PAGE on 12% Tris-glycin gel using Coomassie staining.
   A: 1 ml cell pellet was resuspended in 150 µl BugBuster^{®} solution. 5 µl sample was mixed with 5 µl sample buffer and loaded in each lane. The arrow to the right indicates the recombinant untagged NN_GBS protein, the arrow to the left (in the picture of the gel) indicates GST-NN_GBS fusion protein. Expected size of fusion protein (GST-NN GBS, strain AMJ2746): 68 KDa. Expected size of untagged NN_GBS (strain AMJ3063): 38.4 KDa.
      Lane 1 is loaded with See Blue Plus 2 ladder,
      lane 2: AMJ2746 uninduced,
      lane 3: AMJ2746 0.1 mM IPTG,
      lane 4: AMJ3063 uninduced,
      lane 5: AMJ30630.05 mM IPTG,
      lane 6: AMJ3063 0.1 mM IPTG,
      lane 7: AMJ3063 0.5 mM IPTG.
      Lanes 2-3 contain samples from the GST tagged expression system and lanes 4-7 contain samples from the pET expression system.
      The expected size of the fusion protein (GST-NN BGS) was 68 KDa and the expected size of the untagged NN_GBS protein was 38.4 KDa.
   B: 1 ml cell pellet was resuspended in 150 µl BugBuster solution. 5 µl sample was mixed with 5 µl sample buffer and loaded in each lane. The remaining pellet was boiled in 150 µl sample buffer and 10 µl was loaded in each lane (above). The arrow indicates the NN_GBS control protein (without GST tag). (See EXAMPLE 1)
      Lane 1 is loaded with AMJ2746 uninduced,
      lane 2: AMJ2746 0.1 mM IPTG,
      lane 3: AMJ3063 uninduced,
      lane 4: AMJ30630.05 mM IPTG,
      lane 5: AMJ3063 0.1 mM IPTG,
      lane 6: AMJ3063 0.5 mM IPTG,
      lane 7: 500 ng control protein NN_GBS,
      lane 8: See Blue Plus 2 ladder.
      lanes 1-2 contain samples from the GST tagged expression system and lanes 3-6 contain samples from the pET expression system.

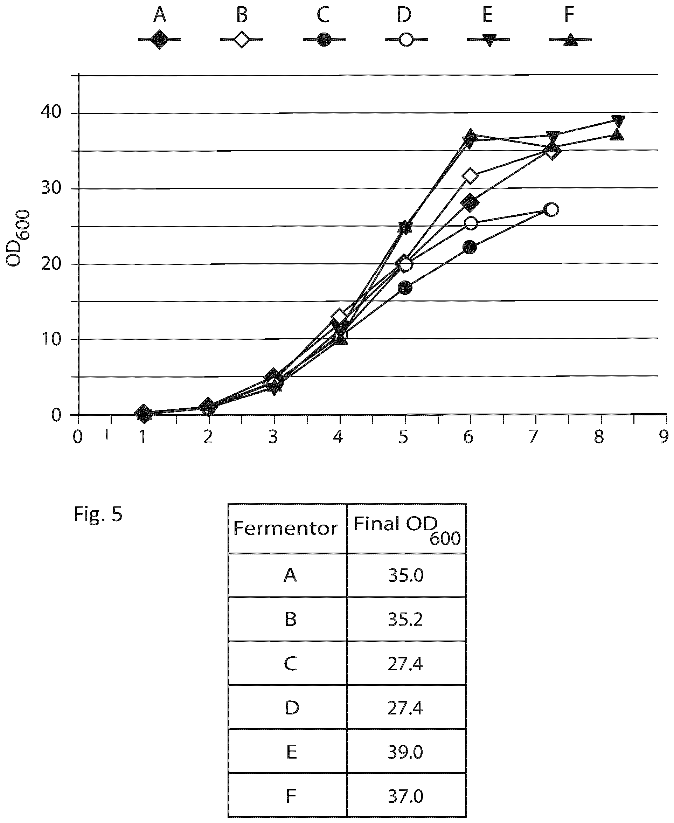

Figure 5 shows growth curves from the batch fermentation experiment in EXAMPLE 1.

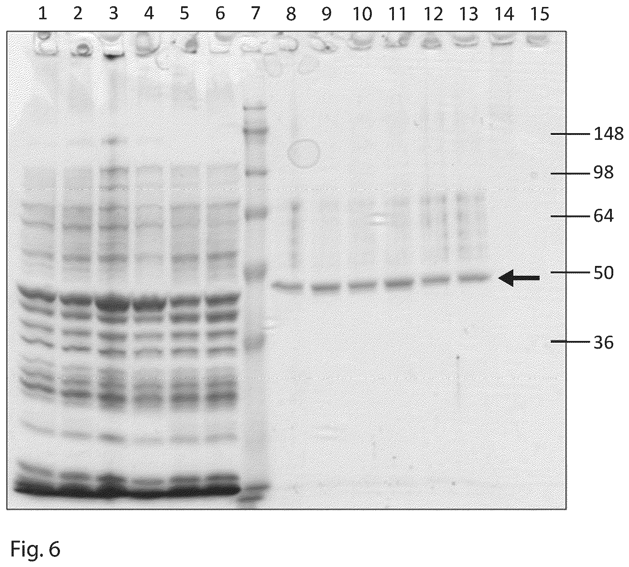

Figure 6 shows an SDS-page gel of insoluble and soluble fractions. Cell pellet from 2 mL culture was solubilized in BugBuster^{®} solution for a final OD of 110 (500-700 µl). The extract was diluted 200-fold in sample buffer and 10 µL was loaded per lane (lanes 8-13). The remaining insoluble pellet was boiled in an equal volume of sample buffer and 5 µl was loaded in each lane (lanes 1-6). The arrow indicates the recombinant untagged NN_GBS protein. (See EXAMPLE 1).
   Lane 1 is loaded with Fermentor A, A4, 4.3h,
   lane 2: Fermentor B, B4, 4.3h, insoluble fraction,
   lane 3: Fermentor C, C4, 4.3h, insoluble fraction,
   lane 4: Fermentor D, D4, 4.3h, insoluble fraction,
   lane 5: Fermentor E, E5, 4.7h, insoluble fraction,
   lane 6: Fermentor F, F5, 4.7h, insoluble fraction,
   lane 7: See Blue Plus 2,
   lane 8: Fermentor A, A4, 4.3h, soluble fraction,
   lane 9: Fermentor B, B4, 4.3h, soluble fraction,
   lane 10: Fermentor C, C4, 4.3h, soluble fraction,
   lane 11: Fermentor D, D4, 4.3h, soluble fraction,
   lane 12: Fermentor E, E5, 4.7h, soluble fraction,
   lane 13: Fermentor F, F5, 4.7h, soluble fraction,
   lane 14: empty,
   lane 15: empty.

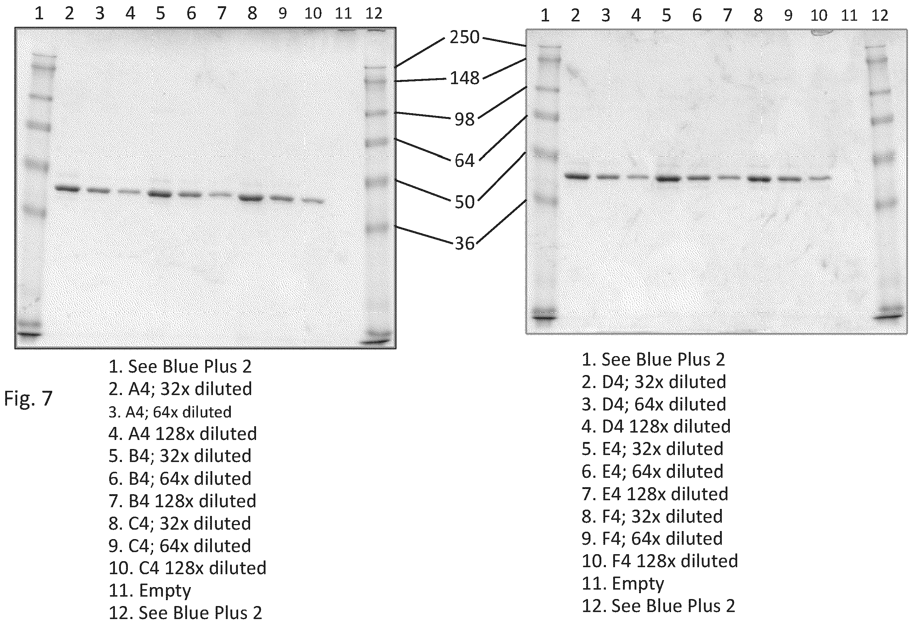

Figure 7 shows SDS-page gels: Cell extracts were prepared (using 200 µl BugBuster^{®} reagent) from end-samples A4, B4, C4, D4, E5 and F5. The same amounts of cells were used (normalized to 1 mL OD600=8). Two-fold dilutions of the cell extracts were prepared and selected dilutions were analyzed by SDS-PAGE. 15 µl were loaded in each lane. (See EXAMPLE 1)

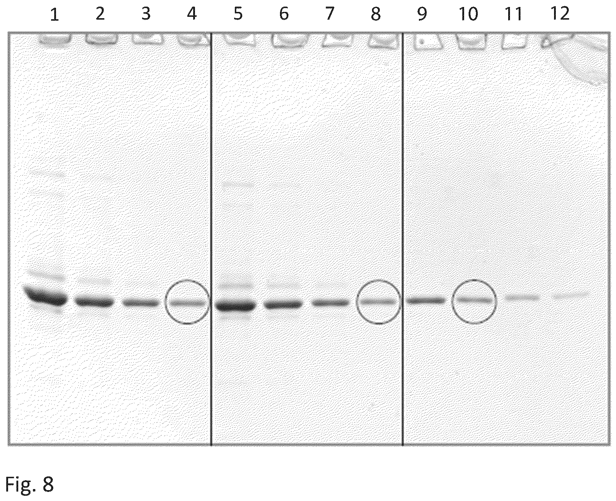

Figure 8 shows the yield of NN_GBS from three different IPTG induction conditions. (See EXAMPLE 1).
   Lane 1 is loaded with B4, 16x diluted (~ 1 µl culture),
   lane 2: B4, 32x diluted (~ 0.5 µl culture),
   lane 3: B4, 64x diluted (~ 0.25 µl culture),
   lane 4: B4, 128x diluted (~ 0.13 µl culture),
   lane 5: E5, 16x diluted (~ 1 µl culture),
   lane 6: E5, 32x diluted (~ 0.5 µl culture),
   lane 7: E5, 64x diluted (~ 0.25 µl culture),
   lane 8: E5, 128x diluted (~ 0.13 µl culture),
   lane 9: Contol NN_GBS, 0.185 µl (0.94 µg acc. Bradford),
   lane 10: Contol NN_GBS, 0.94 µl (0.47 µg acc. Bradford),
   lane 11: Contol NN_GBS, 0.47 µl (0.24 µg acc. Bradford),
   lane 12: Contol NN_GBS, 0.24 µl (0.12 µg acc. Bradford).

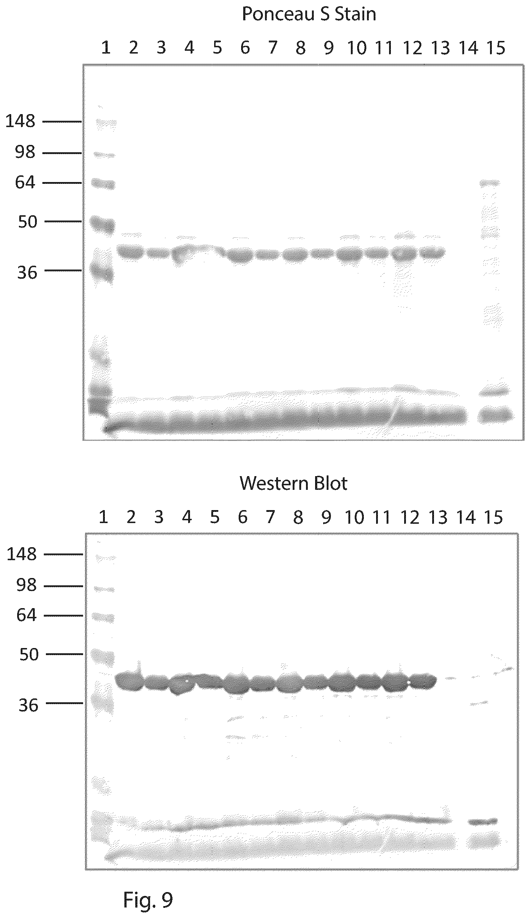

Figure 9 shows the results of a Western blot analysis: Cell extracts were prepared (using 200 µl BugBuster^{®} reagent) from end-samples A4, B4, C4, D4, E5 and F5. The same amounts of cells were used (normalized to 1 mL OD600=8). Two-fold dilutions of the cell extracts were prepared and selected dilutions were analyzed by Western blot analysis using anti-sera against NN_GBS. 15 µl were loaded in each lane. (See EXAMPLE 1).
   Lane 1 is loaded with See Blue plus 2 ladder,
   lane 2: A4 8x diluted,
   lane 3: A4 16x diluted,
   lane 4: B4 8x diluted,
   lane 5: B4 16x diluted,
   lane 6: C4 8x diluted,
   lane 7: C4 16x diluted,
   lane 8: D4 8x diluted,
   lane 9: D4 16x diluted,
   lane 10: E5 8x diluted,
   lane 11: E5 16x diluted,
   lane 12: F5 8x diluted,
   lane 13: F5 16x diluted,
   lane 14: Empty,
   lane 15: E. coli control lysate (negative control).

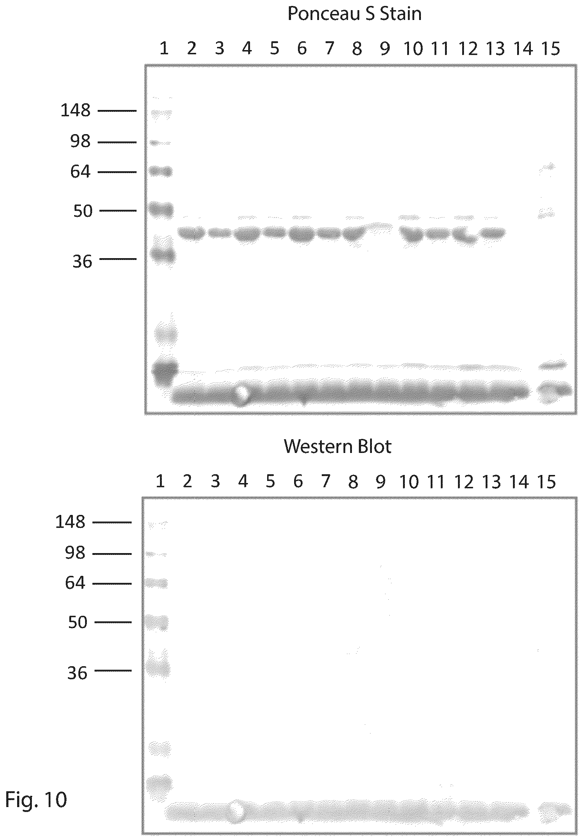

Figure 10 shows the results of a Western blot analysis: Cell extracts were prepared (using 200 µl BugBuster^{®} reagent) from end-samples A4, B4, C4, D4, E5 and F5. The same amounts of cells were used (normalized to 1 mL OD600=8). Two-fold dilutions of the cell extracts were prepared and selected dilutions were analyzed by Western blot analysis using pre-immune antiserum. 15 µl were loaded in each lane (see EXAMPLE 1).
   Lane 1 is loaded with See Blue plus 2 ladder,
   lane 2: A4 8x diluted,
   lane 3: A4 16x diluted,
   lane 4: B4 8x diluted,
   lane 5: B4 16x diluted,
   lane 6: C4 8x diluted,
   lane 7: C4 16x diluted,
   lane 8: D4 8x diluted,
   lane 9: D4 16x diluted,
   lane 10: E5 8x diluted,
   lane 11: E5 16x diluted,
   lane 12: F5 8x diluted,
   lane 13: F5 16x diluted,
   lane 14: Empty,
   lane 15: E. coli control lysate (negative control).

### DETAILED DESCRIPTION

A first aspect of the technology proposed herein concerns a fermentation method for producing a recombinant fusion protein comprising N-terminal regions of group B Streptococcus surface proteins. The method comprises the steps of:
i) growing a host cell, such as an *E. coli* host cell, said host cell comprising a gene encoding said recombinant fusion protein under control of Lacl, in a growth medium;
ii) when the cell density reaches an OD₆₀₀ of from about 3.5 to about 50, such as from about 3.5 to about 30, such as from about 3.5 to about 20, such as from about 3.5 to about 10, such as from about 3.5 to about 8, such as from about 4 to about 5, such as about 4.5, adding IPTG to induce expression of said gene encoding said recombinant fusion protein;
iii) harvesting, concentrating and washing and the grown cells by tangential flow filtration;
iv) disrupting the cells to prepare a homogenate comprising said recombinant fusion protein.

Fermentation is commonly used for large-scale production of microbial products, such as enzymes, pharmaceuticals and proteins and involves growing microbial cells, able to produce the product of interest, in a nutrient-rich growth medium. Fermentation processes for such purposes generally include growing and harvesting the cells, disrupting the cells to release their contents and applying a series of purification steps to obtain the desired product.

The fermentation method of the technology proposed herein enables large scale production of recombinant Streptococcus fusion proteins, fulfilling GMP requirements. Further, the present fermentation method is advantageous as the produced recombinant fusion proteins have less modifications. As explained above, recombinant fusion proteins having less modifications are advantageous for large scale production because they allow the quality control of the produced recombinant proteins to be performed using simpler assays. As an example, it might be acceptable in a laboratory scale production of recombinant fusion proteins to thoroughly investigate the produced recombinant fusion protein using detailed assays and e.g. immunization assays. For a large scale production this is generally not feasible and it is then instead desired to use simpler assays in combination with a stringent production protocol to ascertain that the produced recombinant fusion protein correspond to the desired product. Such an approach is however not possible if the recombinant fusion proteins produced by the large scale production wary widely in e.g. size and hydrophobicity e.g. due to a widely varying extent of modifications, e.g. glycosylation, on the recombinant fusion proteins. Even if the large scale produced recombinant fusion proteins are subjected to e.g. immunological assays and as such as shown to have the desired efficacy, the potential presence of the modifications may lead to unexpected effect when the recombinant fusion protein is formulated into a vaccine and/or administered to a patient. The present fermentation method thus provides a recombinant fusion protein that is suitable for use for immunization. In particular, the fermentation method of the technology proposed herein is suitable to use for the production of recombinant fusion proteins that are to be used to create a protective immune response to Group B Streptococcus.

The recombinant fusion protein referred to according to the technology proposed herein is a fusion protein comprising N-terminal regions of group B Streptococcus surface proteins, optionally linked by a linker. This recombinant fusion protein will be described in further detail in the below.

The method according to the first aspect comprises growing a host cell, such as an *E. coli* host cell, comprising a gene encoding the recombinant fusion protein. *E. coli* host cells are commonly used for the expression of recombinant proteins and the skilled person knows how to select a suitable *E. coli* host. When *E. coli* is used as the host cell, a growth temperature of about 37°C is preferred. An antibiotic may be used in the growth medium to prevent growth of unwanted microorganisms. The growth medium may be any growth medium that supports growth of the host cell but that does not induce premature initiation of expression of the recombinant fusion protein. A preferred growth medium when the host cell is *E. coli* is yeast extract with phytone, peptone and ammonium and potassium salts. However, other types of host cells may also be used, such as other bacterial host cells or yeast cells. Preferably, the host cell is an *E. coli* cell.

Preferably, the gene encoding the recombinant fusion protein is placed on a plasmid cloned into the host cell, even if other means of enabling the host cell to encode the recombinant fusion protein may be used, such as chromosomal integration. Expression of the gene encoding the recombinant fusion protein can be controlled by addition of lactose, allolactose (formed by the conversion of lactose to allolactose by β-galactosidase). For recombinant production, often the allolactose mimic IPTG (isopropyl β-D-1-thiogalactopyranoside) is used instead of lactose to induce expression of genes under control of Lacl. When the OD₆₀₀ reaches a value of from about 3.5 to about 50, such as from about 3.5 to about 30, such as from about 3.5 to about 20, such as from about 3.5 to about 10, such as from about 3.5 to about 8, such as from about 4 to about 5, such as about 4.5 . IPTG is added to the growth medium and the growth is continued while the recombinant fusion protein is expressed. OD₆₀₀ is the optical density of a sample measured at a wavelength of 600 nm in a 1 cm light path, unless otherwise state. OD₆₀₀ is commonly used to follow microbial growth and to determine microbial concentration in liquid samples. Adding IPTG at an OD in the above specified ranges is preferred as it indicates that the number of cells is enough and that the cells are in a state allowing a sufficiently high amount of recombinant fusion protein to be produced in the method.

IPTG is preferably added during the exponential phase of the bacterial growth. IPTG is typically added to the bacterial fermentation culture to a final concentration of from about 0.02 mM to about 1 M, such as from about 0.02 mM to about 0.5 M, such as from about 0.02 mM to about 100 mM, such as from about 0.02 mM to about 50 mM, such as from about 0.02 mM to about 10 mM, such as from about 0.02 mM to about 5 mM, such as from about 0.02 mM to about 2 mM ,such as from about 0.03 to about 0.15 mM, such as from about 0.04 to about 0.15 mM, such as from about 0.05 mM to about 0.15 mM. It is also possible to induce expression of the recombinant fusion protein later during the fermentation, such as at an OD₆₀₀ of from about 10 to about 14, such as about 12. In this case, it may be preferred to use a higher final concentration of IPTG in the bacterial fermentation culture, such as from about 0.3 mM to about 0.5 mM, such as about 0.4 mM.

After induction of expression of the recombinant fusion protein by the addition of IPTG, the growth of the host cell is continued until the desired cell density is obtained. Typically, the fermentation process is ended when the cell density reaches a dry weight of from about 8 to about 12 g/L, such as about 10g/L. The OD₆₀₀ is typically above 30 when the fermentation process is ended, even though it may be lower, such as from 20 and above. Typically, the IPTG is added about 4.5 hours before end of the fermentation process.

When the desired cell density has been reached, the recombinant fusion protein is recovered. The grown cells are harvested, concentrated and washed typically by tangential flow filtration (TFF). TFF is an efficient method for the separation and purification of e.g. cells that utilizes ultrafiltration membranes. TFF is for example often used to concentrate, desalt and/or exchange the buffer of more or less independently on the sample volume. TFF differs from other filtration systems in that the fluid is passed parallel to the membrane, rather than being pushed through a membrane perpendicularly. Thereby, membrane clogging is prevented.

First, the cells are harvested (i.e. separated from the growth medium) and concentrated using TFF. Typically, the cells are concentrated from about 6 to about 12 times, such as from about 8 to about10 times. For example, a 500 kDa hollow fibre may be used for the TFF when the starting volume of the fermented cells is ca 200 L. In this case, the total membrane area may be about 4-5 m², such as about 4.4 m². It is also possible to harvest and concentrate the cells by other means, such as by centrifugation. However, the use of TFF is preferred as it is easy to scale up for production of larger amounts of recombinant fusion proteins.

After the cells have been concentrated, they are washed, preferably in a diafiltration process. Diafiltration is also a TFF process. Any buffer that buffer that has a buffering capacity around the pH of from about 6 to about 9 may be used. For example, 50 mM tris HCl, pH 7.5 may be used in the washing step. Typically, the cells are washed 2 to 7 times, such as about 5 times. Thorough washing is important in order to reduce the amount of host cell proteins in the final homogenate.

The harvesting, concentration and washing steps using TFF is gentle to the recombinant fusion protein, enabling a large amount of undamaged recombinant fusion protein to be recovered. Initial trials using centrifugation for these steps was unsuccessful in providing a sufficiently large amount of undamaged protein. Without wishing to be bound by theory, this may be due to recombinant fusion proteins being sensitive to harsh conditions and mechanical stress. The fermentation method according to the technology proposed herein therefore provides a method for fermenting and recovering a recombinant fusion protein which is gentler than other methods commonly used for production and recovery of recombinant fusion proteins.

After concentration and washing of the cells, the cells are disrupted. This may be performed in any way that disrupts the cell membrane so that the intracellular content of the cells is released. Cell disruption may e.g. be performed by mechanical means, such as by homogenization or bead milling, or by non-mechanical means, such as by heating, osmotic shock, detergents or enzymatically. Preferably, cell disruption according to the technology proposed herein is performed by homogenization, such as high pressure homogenization. The cell concentration when performing the homogenization is not critical but may typically be about 200 g/L. If cell dilution is necessary to reach this cell concentration, this may be performed by diluting the cells in the same buffer they are suspended in after the washing step, typically 50 mM tris HCl, pH 7.5. By disrupting the cells, a homogenate comprising the recombinant fusion protein is obtained. A homogenate is the slurry that results after cell disruption.

The fermentation method of the technology proposed herein may comprise a step v) of clarifying the homogenate obtained in step iv) to obtain a protein solution comprising the recombinant fusion protein. The clarification removes larger particles, such as cell debris and protein aggregates. The clarification is preferably performed by tangential flow filtration and dia-filtration. Firstly, the volume of the homogenate of step iv) may be reduced by TFF, such as reduced by a factor of two. Diafiltration is then performed, typically using the buffer the homogenate is already suspended in. Preferably, tris HCl, pH 7.5 is used also in this step. Typically, 2 to 6 volumes are used for this diafiltration step, such as about 4 volumes. For example, a 500 kDa hollow fibre may be used also for this TFF and diafiltration step. The total membrane area may be about 4-5 m², such as about 4.4 m².

After the clarification step v), the protein solution may be filtered, such as through a 0.2 µm filter. The fermentation method may thus comprise a step vi) of filtering the protein solution obtained in step v), such as through a 0.2 µm filter. The protein solution may then be stored before further purification or use. Preferably, the protein solution is stored at from -15°C to -25°C, such as about -20°C.

The fermentation method of the technology proposed herein may further comprise a pre-cultivation step performed before step i). This pre-cultivation step may be performed by inoculating a smaller volume of stored host cells (comprising the gene of the recombinant fusion protein of interest under control of Lacl), such as from a frozen stock, in a suitable growth medium. The pre-cultivation may be performed in at least two steps, successively increasing the culture volume.

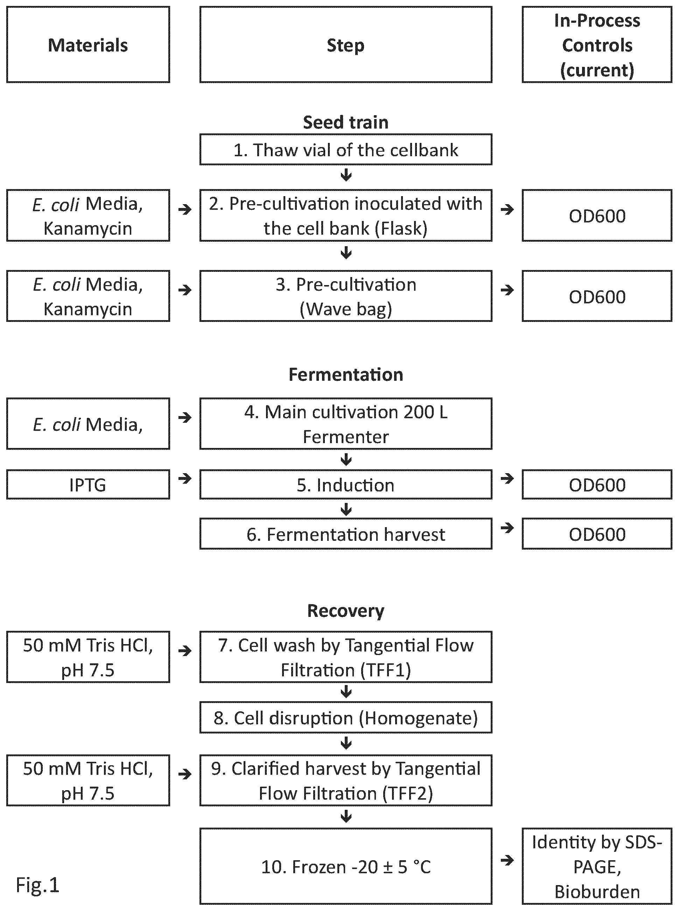

The fermentation method according to the technology proposed herein, including the optional pre-cultivation and clarification steps, is illustrated in a non-limiting way in Fig. 1 and Example 2.

As mentioned above, the host cell may be a bacterial or yeast host that supports replication and expression of the gene for the recombinant fusion protein by IPTG induction. The Lacl repressor is normally placed on a plasmid introduced into the *E. coli* host cell, though so the host cell does not have to be able to express this by itself. The host cell may e.g. be an *E. coli* host cell. Non-limiting examples of *E. coli* host cells that can be used in the technology proposed herein include strain BL21 and its derivatives or the K-12 lineage cells. Preferably, the *E. coli* host cell is BL21.

Preferably, the fermentation method of the technology proposed herein is a batch process. The homogenate produced in step iv) or the protein solution produced in step v) is typically purified further, in particular if the recombinant fusion protein is to be used for immunization or other administration to a subject.

The recombinant fusion protein to be produced by the method of the technology proposed herein comprises a first amino acid sequence having at least 90% sequence identity with the amino acid sequence of the N-terminal region of a first group B Streptococcus surface protein. The first amino acid sequence is fused to, optionally via linker, a second amino acid sequence having at least 90% sequence identity with the amino acid sequence of the N-terminal region of a second group B Streptococcus surface protein. The recombinant fusion protein is preferably an immunogenic fusion protein, i.e. a fusion protein able to elicit a protective immunity against group B Streptococcus, such as by vaccination. The first and second group B Streptococcus surface protein should not be the same. The recombinant fusion protein preferably consists of said first and second group B Streptococcus surface proteins and optionally a linker.

The first and second group B Streptococcus surface protein may be selected from the group consisting of Rib protein, Alp1 protein, Alp2 protein, Alp3 protein, Alp4 protein and AlpC protein, with the proviso that the first and second group B Streptococcus surface proteins are not the same. Alternatively, the recombinant fusion protein comprises at least one amino acid sequence having at least 90% sequence identity with the amino acid sequence of the N-terminal region of the group B Streptococcus surface protein Rib, Alp1, Alp2, Alp3, Alp4 and AlpC.

For example, the recombinant fusion protein may be a fusion protein comprising the N-terminal regions of the Group B Streptococcus Rib and Alpha proteins or the N-terminal regions of the Group B Streptococcus Alp1 and Alp2 proteins.

Preferably, the recombinant fusion protein consists of: a first amino acid sequence part consisting of 170 to 178 amino acids, preferably 174 to 175 amino acids, and having at least 90% sequence identity with the amino acid sequence shown in SEQ ID NO: 1 and a second amino acid sequence part consisting of 165 to 174 amino acids, preferably 169 to 170 amino acids, and having at least 90% sequence identity with the amino acid sequence shown in SEQ ID NO: 2 and optionally a linker amino acid sequence part consisting of 1 to 20 amino acids and separating the first amino acid sequence part from the second amino acid part, and wherein the fusion protein preferably consists of 335 to 372 amino acids, preferably 343 to 353 amino acids, more preferably 343 to 347 amino acids. Further, details of such a recombinant fusion protein can be found in WO2022/207657.

The recombinant fusion protein may preferably have an amino acid sequence of SEQ ID NO: 3, or an amino acid sequence having at least 90%, at least 92%, at least 94%, at least 96%, at least 98%, at least 99%, or 100% identity thereto. This recombinant fusion is a fusion protein between the N-terminal regions of Rib and AlpC with an EF linker, herein also denoted GBS-NN.

Alternatively, the recombinant fusion protein consists of: a first amino acid sequence part consisting of 169 to 177 amino acids, preferably 172 to 173 amino acids, and having at least 90% sequence identity with the amino acid sequence shown in SEQ ID NO: 4 and a second amino acid sequence part consisting of 168 to 178 amino acids, preferably 171 to 172 amino acids, and having at least 90% sequence identity with the amino acid sequence shown in SEQ ID NO: 5 and optionally a linker amino acid sequence part consisting of 1 to 20 amino acids and separating the first amino acid sequence part from the second amino acid part, and wherein the fusion protein preferably consists of 338 to 375 amino acids, preferably 343 to 353 amino acids, more preferably 343 to 347 amino acids. Further, details of such a recombinant fusion protein can be found in WO2022/207657.

The recombinant fusion protein may preferably have an amino acid sequence of SEQ ID NO: 6 or an amino acid sequence having at least 90%, at least 92%, at least 94%, at least 96%, at least 98%, at least 99%, or 100% identity thereto. This recombinant fusion protein is a fusion protein between the N-terminal regions of Alp1 and Alp2 with an EF linker, herein also denoted GBS-NN2.

The first and second amino acid sequences may preferably be separated by a linker. This linker is typically 1-20 amino acids long, preferably 1 to 10 amino acids, preferably 2 to 5 amino acids, more preferably 2 amino acids, and most preferably the linker consist of the amino acid sequence EF. The function of the linker is to join the first and second group B Streptococcus N-terminal regions. The linker is typically a linker that is not cleavable.

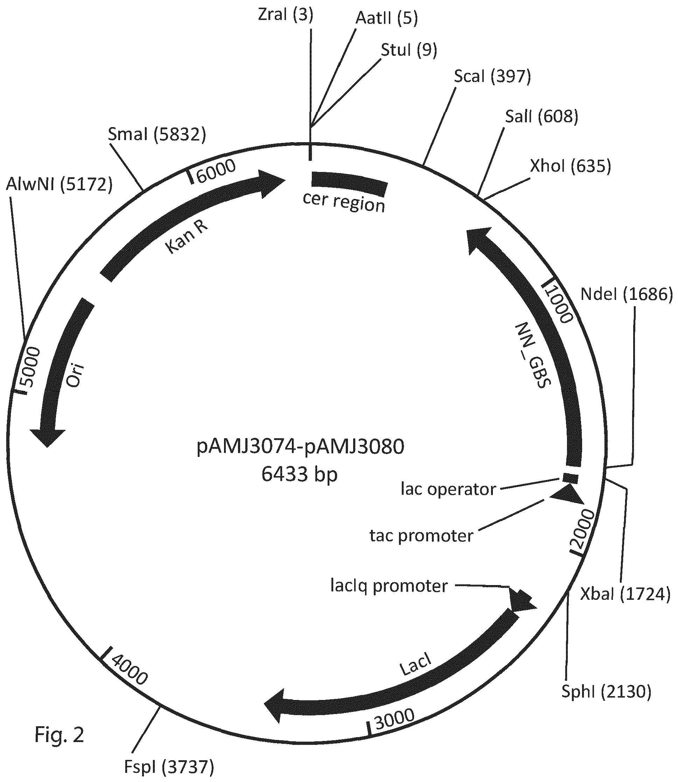

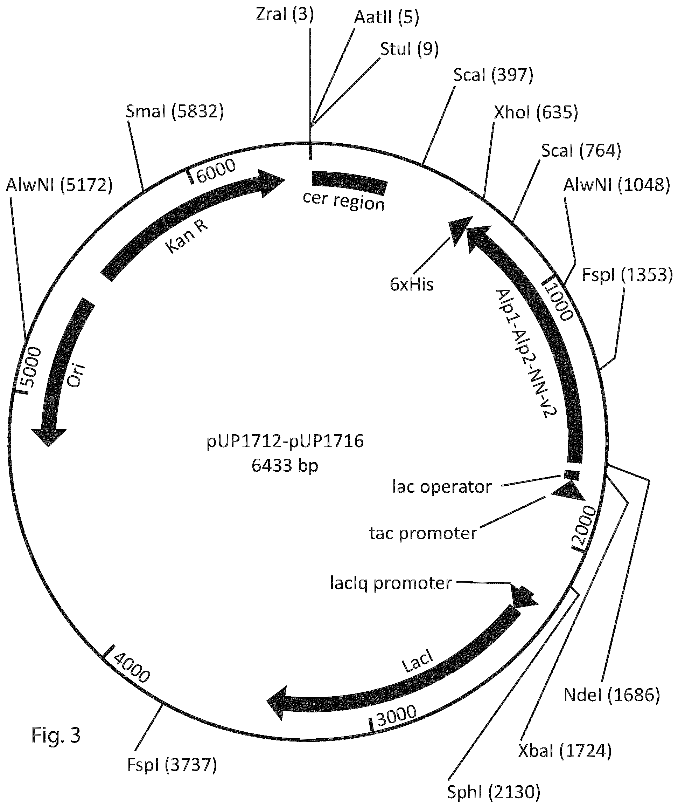

Plasmid maps of two preferred plasmids for expression of the recombinant fusion proteins GBS-NN (SEQ ID NO: 3) and GBS-NN2 (SEQ ID NO: 6) according to the technology proposed herein are illustrated in Figs. 2 and 3, respectively. In the context of the present document, GBS-NN may also be denoted NN-GBS and GBS-NN2 may also be denoted NN2-GBS. GBS-NN and GBS-NN2 are preferably encoded by the nucleic acid sequences of SEQ ID NO: 7 and SEQ ID NO: 8, respectively. The technology proposed herein is therefore also directed to a plasmid encoding a recombinant fusion protein, said plasmid comprising a nucleic acid sequence for expression of GBS-NN encoded by SEQ ID NO: 7, such as the plasmid denoted pAMJ3074-pAMJ3080 which is illustrated in Fig. 2. The technology proposed herein is therefore also directed to a plasmid encoding a recombinant fusion protein, said plasmid comprising a nucleic acid sequence for expression of GBS-NN2 encoded by SEQ ID NO: 8, such as the plasmid denoted pUP1712-pUP1716 which is illustrated in Fig. 3.

In a second aspect, the technology proposed herein is directed to a recombinant fusion protein as defined herein, wherein said recombinant fusion protein has been obtained or is obtainable by a method according to the first aspect of the technology proposed herein.

The technology proposed herein is in a third aspect directed to a fermentation method for producing a recombinant fusion protein with less modifications, wherein said fermentation method comprises the steps of the fermentation method of the first aspect of the technology proposed herein.

The technology proposed herein is in a fourth aspect directed to fermentation method for reducing the number of modifications of a recombinant fusion protein, said fermentation method comprising the steps of the fermentation method of the first aspect of the technology proposed herein.

Preferably, the technology proposed herein is directed to a fermentation method for producing a recombinant fusion protein according to SEQ ID NO: 3 or SEQ ID NO: 6, or an amino acid sequence having at least 90%, at least 92%, at least 94%, at least 96%, at least 98%, at least 99%, or 100% identity to one of these sequences,
said method comprising the steps of:
i) growing an *E. coli* host cell, preferably *E. coli* BL21, said *E. coli* host cell comprising a gene encoding said recombinant fusion protein under control of Lacl in a growth medium, such as comprising a plasmid as depicted in Fig. 1 or 2;
ii) when the cell density reaches an OD₆₀₀ of from about 3.5 to about 6, such as from about 4 to about 5.5, such as from about 4 to about 5, such from about 4.3 to about 4.8, such as about 4.5, adding IPTG to induce expression of said gene encoding said recombinant fusion protein;
iii) harvesting, concentrating and washing the grown cells by tangential flow filtration;
iv) disrupting the cells to prepare a homogenate comprising said recombinant fusion protein;
v) clarifying said homogenate of step iv), by tangential flow filtration and dia-filtration to obtain a protein solution; and
vi) filtering the protein solution of step v), such as through a 0.2 µm filter.

By performing the method for producing a recombinant fusion protein according to SEQ ID NO: 3 or SEQ ID NO: 6 in accordance with this method, the resulting product will be very similar between different batches, which is advantageous for a recombinant fusion protein that is to be used for immunization.

Whenever an amino or nucleic acid sequence is referred to herein, this also includes a variant of said amino or nucleic acid sequence, wherein said variant has a sequence identity of at least 95%, such as at least 96%, such as at least 97%, such as at least 98%, such as at least 99% or 100% identity to the sequence referred to. According to the technology proposed herein, sequence identity is calculated in the following manner. By an amino or nucleic acid sequence having a sequence with at least, for example 95%, identity to a reference sequence, is intended that the sequence is identical to the reference sequence except that the sequence may include up to 5 amino or nucleic acid differences per each 100 amino or nucleic acids of the reference sequence. In other words, to obtain an amino or nucleic acid sequence having a sequence identity of at least 95%, up to 5% of the amino or nucleic acids in the reference sequence may be deleted or substituted with another amino or nucleic acid, or a number of amino or nucleic acids up to 5% of the total number of amino or nucleic acids in the reference sequence may be inserted into the reference sequence. If the two sequences to be compared are not of equal length, they must be aligned to best possible fit, and the sequence identity is calculated over the length of the reference sequence. These amino or nucleic acid changes of the reference sequence may occur at the amino and/or carboxy terminal positions for an amino acid sequence or at the 5' and/or 3' end for a nucleic acid sequence of a reference amino or nucleic acid sequence or anywhere between those terminal positions, interspersed either individually within the reference sequence or in one or more contiguous groups within the reference sequence.

### SEQUENCES

(methionine in the N-terminal end is always optional and not to be included when sequence identities are calculated):
SEQ ID NO: 1 (N-terminal region of Rib) 
MAEVISGSAVTLNTNMTKNVQNGRAYIDLYDVKNGKIDPLQLITLNSPDLKAQYVIRQGGN
YFTQPSELTTVGAASINYTVLKTDGSPHTKPDGQVDIINVSLTIYNSSALRDKIDEVKKKAE
DPKWDEGSRDKVLISLDDIKTDIDNNPKTQSDIANKITEVTNLEKILVPRIP
SEQ ID NO: 2 (N-terminal region of AlpC) 
STIPGSAATLNTSITKNIQNGNAYIDLYDVKLGKIDPLQLIVLEQGFTAKYVFRQGTKYYGD
VSQLQSTGRASLTYNIFGEDGLPHVKTDGQIDIVSVALTIYDSTTLRDKIEEVRTNANDPK
WTEESRTEVLTGLDTIKTDIDNNPKTQTDIDSKIVEVNELEKLLVLS
SEQ ID NO: 3 (GBS-NN): 
MAEVISGSAVTLNTNMTKNVQNGRAYIDLYDVKNGKIDPLQLITLNSPDLKAQYVIRQGGN
YFTQPSELTTVGAASINYTVLKTDGSPHTKPDGQVDIINVSLTIYNSSALRDKIDEVKKKAE
DPKWDEGSRDKVLISLDDIKTDIDNNPKTQSDIANKITEVTNLEKILVPRIPEFSTIPGSAAT
LNTSITKNIQNGNAYIDLYDVKLGKIDPLQLIVLEQGFTAKYVFRQGTKYYGDVSQLQSTG
RASLTYNIFGEDGLPHVKTDGQIDIVSVALTIYDSTTLRDKIEEVRTNANDPKWTEESRTE
VLTGLDTIKTDIDNNPKTQTDIDSKIVEVNELEKLLVLS
SEQ ID NO: 4 (N-terminal region of Alp1) 
MAEVISGSAATLNSALVKNVSGGKAYIDIYDVKNGKIDPLNLIVLTPSNYSANYYIKQGGRI
FTSVNQLQTPGTATITYNILDENGNPYTKSDGQIDIVSLVTTVYDTTELRNNINKVIENAND
PKWSDDSRKDVLSKIEVIKNDIDNNPKTQSDIDNKIVEVNELEKLLVLP
SEQ ID NO: 5 (N-terminal region of Alp2) 
STIPGSAATLNTSITKNIQNGNAYIDLYDVKNGLIDPQNLIVLNPSSYSANYYIKQGAKYYS
NPSEITTTGSATITFNILDETGNPHKKADGQIDIVSVNLTIYDSTALRNRIDEVINNANDPKW
SDGSRDEVLTGLEKIKKDIDNNPKTQIDIDNKINEVNEIEKLLVVSL
SEQ ID NO: 6 (GBS-NN2, N-terminal Met removed post-translationally): 
MAEVISGSAATLNSALVKNVSGGKAYIDIYDVKNGKIDPLNLIVLTPSNYSANYYIKQGGRI
FTSVNQLQTPGTATITYNILDENGNPYTKSDGQIDIVSLVTTVYDTTELRNNINKVIENAND
PKWSDDSRKDVLSKIEVIKNDIDNNPKTQSDIDNKIVEVNELEKLLVLPEFSTIPGSAATLN
TSITKNIQNGNAYIDLYDVKNGLIDPQNLIVLNPSSYSANYYIKQGAKYYSNPSEITTTGSAT
ITFNILDETGNPHKKADGQIDIVSVNLTIYDSTALRNRIDEVINNANDPKWSDGSRDEVLTG
 LEKIKKDIDNNPKTQIDIDNKINEVNEIEKLLVVSL
SEQ ID NO: 7 (codon optimized GBS-NN gene): 
ATGGCCGAAG TTATTAGCGG TAGCGCAGTT ACCCTGAATA CCAATATGAC
CAAAAATGTG CAGAATGGCC GTGCCTATAT TGATCTGTAT GATGTGAAAA
ATGGCAAAAT CGATCCGCTG CAGCTGATTA CCCTGAATTC TCCGGATCTG
AAAGCACAGT ATGTTATTCG TCAGGGTGGT AACTATTTTA CCCAGCCGAG
CGAACTGACC ACCGTTGGTG CAGCAAGCAT TAACTATACC GTTCTGAAAA
CCGATGGTAG TCCGCATACC AAACCGGATG GTCAGGTTGA TATTATCAAT
GTTAGCCTGA CCATCTATAA TAGCAGCGCA CTGCGTGATA AAATTGATGA
AGTGAAAAAG AAAGCCGAAG ATCCGAAATG GGATGAAGGT AGCCGTGATA
AAGTTCTGAT TAGCCTGGAT GATATCAAAA CCGATATTGA TAACAACCCG
AAAACCCAGA GCGATATTGC AAACAAAATT ACCGAAGTGA CCAATCTGGA
AAAAATTCTG GTTCCGCGTA TTCCGGAATT TAGCACCATT CCGGGTAGCG
CAGCAACACT GAATACAAGC ATTACCAAAA ACATTCAGAA CGGCAACGCA
TATATCGATC TGTACGACGT TAAACTGGGT AAAATTGATC CTCTGCAACT
GATCGTTCTG GAACAGGGTT TTACCGCAAA ATATGTGTTT CGTCAGGGCA
CCAAATACTA TGGTGATGTT AGCCAGCTGC AGAGCACCGG TCGTGCAAGC
CTGACCTATA ACATTTTTGG TGAAGATGGT CTGCCGCATG TTAAAACCGA
TGGCCAGATT GATATCGTTA GCGTTGCACT GACGATTTAT GATAGCACCA
CCCTGCGCGA TAAAATCGAA GAAGTTCGTA CCAACGCAAA CGATCCTAAA
TGGACCGAAG AAAGCCGTAC CGAAGTTCTG ACCGGTCTGG ATACCATTAA
AACGGATATC GACAATAATC CTAAAACGCA GACAGATATT GATAGCAAAA
TTGTGGAAGT GAATGAACTGGAAAAACTGC TGGTTCTGAG CTAATAA
SEQ ID NO: 8 (codon optimized GBS-NN2 gene): 
ATGGCCGAAGTTATTAGCGGTAGCGCAGCAACCCTGAATAGCGCACTGGTTAAAAAT
GTTAGCGGTGGCAAAGCCTATATCGACATCTATGATGTGAAAAACGGCAAAATTGAT
CCGCTGAATCTGATTGTTCTGACCCCGAGCAATTATAGCGCCAACTATTATATCAAAC
AGGGTGGTCGTATTTTCACCAGCGTTAATCAGCTGCAGACACCGGGTACAGCAACCA
TTACCTATAACATTCTGGATGAAAATGGCAACCCGTATACCAAAAGTGATGGCCAGAT
TGATATTGTTAGCCTGGTTACCACCGTTTATGATACCACCGAACTGCGCAATAACATC
AACAAAGTTATTGAGAATGCCAACGACCCGAAATGGTCAGATGATAGCCGTAAAGAT
GTTCTGAGCAAAATCGAGGTGATCAAAAACGATATTGATAACAACCCGAAAACCCAG
AGCGATATCGACAACAAAATTGTGGAAGTGAACGAGCTGGAAAAACTGCTGGTTCTG
CCGGAATTTAGCACCATTCCGGGTTCAGCAGCCACACTGAATACCAGCATTACCAAA
AACATTCAGAATGGCAACGCCTACATTGATCTGTACGATGTAAAAAATGGTCTGATCG
 
ATCCGCAGAACCTGATCGTGCTGAATCCGAGCAGCTATTCAGCCAATTATTATATTAA
ACAAGGCGCAAAATACTATAGCAACCCGAGCGAAATTACCACCACCGGTAGCGCCA
CCATTACGTTTAATATCCTGGACGAAACCGGTAACCCGCATAAAAAGCAGATGGTC
AAATTGATATCGTGAGCGTTAACCTGACGATTTATGATAGCACAGCCCTGCGTAATCG
TATTGATGAAGTGATTAATAACGCGAATGATCCTAAATGGTCCGATGGTAGTCGTGAT
GAAGTACTGACCGGTCTGGAAAAATCAAAAAGACATCGACAATAATCCGAAAACG
CAGATTGACATTGACAATAAAATCAACGAGGTGAACGAGATCGAGAAACTGCTGGTA
GTTAGCCTGTAATAA
SEQ ID NO: 9 (Alp4N):

VVEGSAATLNTAMTKNMQNGNAYIDIYDVKLGKIDPLQLIKLEPGYTAIYYITQGSKVYA
NVSELQTPGAAKVNYRIQTSDGSDHIKSDGQLDSVNISLTVYD

### EXPERIMENTAL SECTION

The fermentation method of the technology proposed herein is in this experimental section exemplified via the production of the two recombinant fusion proteins denoted GBS-NN (SEQ ID NO: 3) and GBS-NN2 (SEQ ID NO: 6).

### EXAMPLE 1: Optimisation of IPTG induction

The aim of this experiment is to test what conditions are suitable for IPTG induction of expression of a recombinant GBS-NN fusion protein.

An un-tagged NN_GBS gene (codon optimized) was synthesized by GeneArt and cloned into the pET expression system (vector pET-26b(+)) and into the E-c expression system developed by Bioneer (vector pAMJ2630, final plasmid construct denoted pAMJ3074-pAMJ3080, see Fig. 2). The E-c vector is composed of genetic elements that are free of IPR.

Constructs were introduced into the expression host BL21 (DE3) (the pET-26b(+) construct, strain with vector denoted AMJ3063) and into BL21 (the E-c construct, strain with vector denoted AMJ3080).

### Shake flask expression

The strains were grown overnight in LB medium containing appropriate antibiotics (kanamycin). The overnight cultures were diluted 100 times in fresh LB medium (with antibiotics) and grown at 37°C at 250 rpm until OD₆₀₀=0.6-0.7. The cultures were then split and induced using different concentrations of IPTG. Two induction experiments took place.

### Induction experiment 1

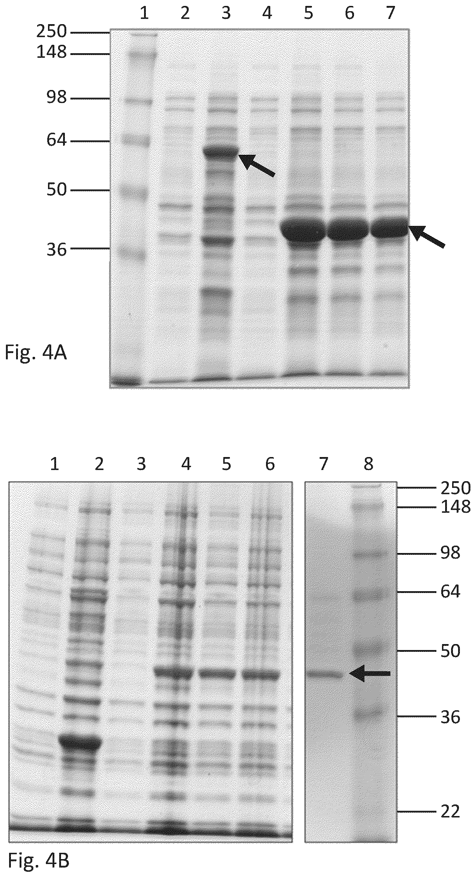

In the first induction experiment with AMJ3063 (pET expression system), strain AMJ2746, which produces the GST-tagged NN_GBS from the expression vector pGEX-6P-2 (AmpR) in *E. coli* strain BL21 was also included. The following IPTG concentrations were tested for AMJ3063: 0.05, 0.1 and 0.5 mM, while only 0.1 mM was used for induction in strain AMJ2746. Final cell densities (OD₆₀₀) around 3.5-4.0 were obtained in both strains. Culture samples of 1 ml were harvested 4 hours after induction. The soluble fractions were obtained using 150 µl BugBuster^{®} (Novagen) while the insoluble fractions were recovered by boiling the pellets remaining after the BugBuster^{®} treatment in 150 µl sample buffer. The obtained samples were analysed by SDS-PAGE on a 12% Tris-glycin gel using Coomassie staining (Figs. 4A and B). Most NN_GBS material is present in the soluble fractions (Fig. 4A) and only very little NN_GBS material is present in the insoluble fractions (Fig. 4B). The production level was significantly higher in the pET expression system compared to the GST tagged vector system. No expression was observed before IPTG induction. The expression level from the pET expression system was slightly higher at the lower IPTG concentration.

### Induction experiment 2

This experiment included AMJ3063 (pET expression system) and AMJ3080 (E-c expression system). The following IPTG concentrations were tested for AMJ3080: 0.05, 0.1 and 0.5 mM, while only 0.05 mM was used for induction in strain AMJ3063. Final cell densities (OD₆₀₀) around 3.5-4.0 were obtained in both strains when using 0.05 and 0.1 mM IPTG, while growth was inhibited in strain AMJ3080 when using 0.5 mM IPTG, resulting in OD₆₀₀ around 2.7. 1 ml culture samples were harvested 4 hours after induction. Soluble fractions and insoluble fractions were obtained as described above and analysed by SDS-PAGE using Coomassie staining. Again, most NN_GBS material was present in the soluble fractions (data not shown) and only very little NN_GBS material in the insoluble fractions (data not shown). The production level in the E-c system is at the same level as in the pET expression system. Also, in the case of the E-c system it seemed that the highest expression was obtained using the low concentration of IPTG (0.05 mM). No expression was observed before IPTG induction.

Based on the shake flask studies it was decided to use strain AMJ3080 (E-c system) for production of NN_GBS material in six 1 L bioreactor fermentations.

### Protein expression and localization of gene product

### Experimental setup

The fermentors used were Applikon glass dished bottom vessels with a total volume of 2 L equipped with two Rushton impellers. In batch fermentations the working volume was 800 mL. The medium used is called Ec-004, which is an animal-free enriched medium containing 2% yeast extract, 2% soy peptone and 1% glucose. 25 mg/l of kanamycin was included in the growth medium for AMJ3080.

A pre-culture of strain AMJ3080 was prepared by growth over-night in LB medium with kanamycin. 800 ml medium was inoculated with 20 mL of pre-culture with an OD₆₀₀ of approximately 3.5. During the fermentation, pH was maintained at pH 6.5 by addition of 2 M NaOH and 2 M H₃PO₄. Oxygen was supplied by sparging with air at 1 L/min, and pO₂ was maintained at 30% by increasing the agitation rate. The temperature was kept at 37°C, also during the production phase. Growth was followed by OD₆₀₀ readings (off-line). Production of the heterologous protein NN_GBS was induced by addition of IPTG. 2 ml samples were harvested, and the cell pellets were stored at -20°C for analysis of NN_GBS production during the fermentation. The cultures (approximately 700 ml) were harvested at the end of the fermentation. The cell pellets were resuspended in ice-cold 50 mM Tris-HCl pH 7.5, 150 mM NaCl buffer, transferred to 50 ml tubes, harvested again and subsequently stored at -20°C for later extraction of NN_GBS using high pressure homogenization.

### Batch fermentation experiment

Three different induction conditions were tested for AMJ3080 and each condition was performed in duplicate. The following induction conditions were used: 0.1 mM IPTG at OD₆₀₀=4; 0.4 mM IPTG at OD₆₀₀=4; 0.4 mM IPTG at OD₆₀₀=12.

The final OD₆₀₀ is shown in Table 1; the growth curves are shown in Fig. 5.

**Table 1. Overview of fermentation experiment**

| **Fermentor** | **Induction at** | **IPTG concentration** | **Induction period** | **Final OD₆₀₀** |
|---|---|---|---|---|
| A | OD₆₀₀ = 4 | 0.1 mM | 4.5 hours | 35 |
| B | OD₆₀₀ = 4 | 0.1 mM | 4.5 hours | 35 |
| C | OD₆₀₀ = 4 | 0.4 mM | 4.5 hours | 27 |
| D | OD₆₀₀ = 4 | 0.4 mM | 4.5 hours | 27 |
| E | OD₆₀₀ = 12 | 0.4 mM | 4.7 hours | 39 |
| F | OD₆₀₀ = 12 | 0.4 mM | 4.7 hours | 37 |

Batch fermentations at 37°C in 800 mL of an enriched medium (Ec-004) containing 20 g/L yeast extract, 20 g/L Phytone Peptone and 10 g/L glucose at pH 6.5.

NN_GBS production was analysed in cells from 2 mL samples taken during the fermentation. The soluble fraction was extracted using BugBuster^{®} reagent and the insoluble fraction was solubilized by boiling the pellet remaining after the BugBuster^{®} extraction. The volumes of BugBuster^{®} reagent and sample buffer were adjusted to correspond to a cell suspension of OD600 = 110. 5 µL of the soluble BugBuster^{®} extract and 10 µL of the insoluble fraction were loaded for SDS-PAGE. A heavy band in the soluble fraction corresponding to NN_GBS is clearly visible in all lanes after IPTG induction, independent of the applied induction condition (data not shown). Due to the high expression of NN_GBS, the SDS-PAGE gel was heavily overloaded. Therefore, an extra gel was prepared after 200-fold dilution of the soluble fractions (Fig. 6).

### Estimation of NN_GBS in bioreactors

In order to estimate the production of NN_GBS, new BugBuster^{®} extracts were prepared from smaller portions of culture sample, and the extracts were diluted as described below and in Table 2.

A frozen cell pellet from 2 ml harvested culture was resuspended in 2 ml 0.9% NaCl. A sample corresponding to 1 mL of OD₆₀₀ = 8 (e.g. 229 µl from an OD 35 cell suspension in the case of sample A4) was transferred to a fresh Eppendorf tube and harvested by centrifugation. The cell pellet was then extracted in 200 µl BugBuster^{®} reagent. 20 µl of the soluble extract was diluted 4 times by mixing with 20 µl PBS and 40 µl 4x SDS-PAGE sample buffer. A series of two-fold dilutions down to 128 fold was made by mixing 40 µL with 40 µL of 2x SDS-PAGE sample buffer (4x SDS-PAGE mixed 1:1 with PBS). 15 µl of each of the 32-, 64- and 128-fold dilutions was loaded for SDS PAGE.

**Table 2. Volumes of culture corresponding to each loaded sample.**

| **Fermentor culture sample** | **OD₆₀₀** | **µl culture* for BB extraction in 200 µL** | **µL culture per µL undiluted extract** | **µL culture per 15 µL of 32x dilution** | **µL culture per 15 µL of 64x dilution** | **µL culture per 15 µL of 128x dilution** |
|---|---|---|---|---|---|---|
| A4 | 35.0 | 229 | 1.14 | 0.54 | 0.27 | 0.134 |
| B4 | 35.2 | 227 | 1.14 | 0.53 | 0.27 | 0.133 |
| C4 | 27.4 | 292 | 1.46 | 0.68 | 0.34 | 0.171 |
| D4 | 27.4 | 292 | 1.46 | 0.68 | 0.34 | 0.171 |
| E5 | 39.0 | 205 | 1.03 | 0.48 | 0.24 | 0.120 |
| F5 | 37.0 | 216 | 1.08 | 0.51 | 0.25 | 0.127 |
| * This amount of culture corresponds to 1 mL OD₆₀₀ 8 | | | | | | |

All end-of-process samples (A4, B4, C4, D4, E5, and F5) were analysed as described above. Fig. 7 shows the Coomassie Brilliant Blue stained gel. The protein band intensities were very similar in the same dilution steps from all cultures, indicating that the yield of NN_GBS per cell mass did not vary significantly among the three different induction conditions that were tested. The cell density, and thereby the NN_GBS yield per volume, was highest in fermenters E & F, nearly the same in A & B, and 25-30% lower C & D. Samples B4 and E5 were selected for a semi-quantitative analysis using the previously purified NN_GBS batch as a reference protein. This reference is 0.5 mg/ml (Bradford analysis) or 0.185 mg/ml (amino acid analysis determination). A two-fold dilution series of the reference protein was loaded corresponding to 0.94 µg, 0.47 µg, 0.24 µg, 0.12 µg of NN_GBS (Based on Bradford analysis). By visual inspection it was assessed which dilution of B4 and E5 that matched a certain amount of reference NN_GBS protein based on band intensity (Fig. 8). By this visual inspection it was judged that 0.12-0.13 µl culture (B4 and E5) corresponds to 0.47 µg of the reference protein (by Bradford) or 0.17 µg (by amino acid analysis) and therefore the concentration of NN_GBS in the bioreactor was estimated to be ca 3.6 g/L (Bradford) or ca 1.3 g/L (amino acid analysis).

### Western blot analysis

In order to verify the identity of the recombinant protein a Western blot analysis was performed using two different antisera (NN_GBS antiserum and pre-immune antiserum provided by, Lund University). BugBuster^{®} extracts of end-of-process samples from each bioreactor, prepared and diluted 8- and 16-fold and analysed as described above. Furthermore, a negative control for unspecific binding was performed by including an extract from an *E. coli* strain that does not produce NN_GBS. Standard protocols for Western blotting were used. The primary NN_GBS antiserum was diluted 100.000 times and the pre-immune antiserum was diluted 10.000 times while the secondary antibody alkaline phosphatase conjugated anti-rabbit IgG (KPL, catalogue no. 075-1506) was diluted 1.000 times. Both membranes were developed using the BCIP/NBT ready to use solution. Western blots are shown on Fig. 9 and 10. The use of the specific NN_GBS antisera demonstrated very specific recognition (with no background) of NN_GBS in all analysed samples. There was no unspecific recognition of *E. coli* proteins and no degradation of NN_GBS was visible. The use of the pre-immune antiserum showed as expected no recognition of the NN_GBS or any protein which was present in the *E. coli* extract.

### EXAMPLE 2: Manufacturing process

The aim of this example is to provide an exemplary way of performing the fermentation method of the technology proposed herein. In short, the manufacturing process is performed by cultivation of a recombinant *E. coli* host strain (BL21) able to express GBS-NN (plasmid pAMJ3074-pAMJ3080, Fig. 3) or GBS-NN2 (pUP1712-pUP1716, Fig. 4). The upstream manufacturing process flow comprises a seed train (Steps 1 to 3, pre-cultivation); fermentation and harvest (Steps 4 to 6); cell wash, cell disruption by homogenization, harvest and clarification (Steps 7 to 9); and storage at -20 ± 5°C (Step 10). The manufacturing flow scheme for GBS-NN and GBS-NN2 is provided in Fig. 1.

### Pre-Cultivation - Seed Train (Fig. 1. Step 1 to 3)

A vial from a cell bank, was thawed, and the cells were pre-cultivated in 2 steps. The first pre-cultivation step consisted of the inoculation of 125 µL of the cell bank in 250 mL of a medium in a 2 L flask. The second pre-cultivation step was the inoculation of the 250 mL in 4.65 L of the medium in a 20 L wave bag. LB APS medium supplemented with 25 µm kanamycin was used as the medium for the precultivation steps and the growth temperature was 37 °C.

### Main Cultivation in 200 L Reactor (Fig. 1: Step 4 to 6)

The 200 L fermentation process was started with an initial volume of 200 L. The biomass was grown exponentially during the batch process to approximately 10 g/L dry weight over 6 to 8 h. The production of GBS-NN or GBS-NN2 was initiated by induction approximately 4.5 hours before the end of the fermentation process when the acceptance criterion for induction was reached (OD₆₀₀ of about 4.5), by adding Isopropyl β-d-1-thiogalactopyranoside (IPTG) to a final concentration of 0.1 mM. The temperature, pH, and dissolved oxygen tension were controlled at pre-determined set points throughout the fermentation process. The temperature was 37°C.

### Cell Harvest, concentration and wash (Fig. 1: Step 6 to 7)

The cells were harvested, concentrated, and washed by tangential flow filtration (TFF1) using a 500 kDa hollow fibre with a total membrane area of 4.4 m². The cells were concentrated until the total volume was reduced by a factor of 10. Dia-filtration was performed (five volumes) using 50 mM tris HCl, pH 7.5. The concentrated and washed cell mass was processed further immediately.

### Cell Disruption and Protein Release (Fig. 1: Step 8)

The concentrated cell mass was further processed by disrupting the cells to produce a cell homogenate. The cells were diluted with 50 mM tris HCl, pH 7.5, to final concentration of 200 g/L. The cells were disrupted by one cycle of high pressure homogenisation at 1.25 kbar.

### Harvest Clarification to Produce the Homogenate Storage (Fig. 1: Step 9 to 10)

After cell disruption, the cell homogenate was directly clarified using TFF (TFF2) 500 kDa hollow fibre, total membrane area 4.4 m². Concentration was performed until the volume was reduced by a factor of two. Diafiltration was performed (four volumes) using 50 mM tris HCl, pH 7.5. Approximately 125 L of permeate (i.e. protein solution) was collected, filtered through a 0.2 µm filter into a storage tank, divided into approximately 12 L aliquots and stored at -20 ± 5°C.

In the below Table 3, details regarding process solutions and buffer compositions used in this example can be found.

**Table 3: Process solutions and buffer compositions**

| **Use** | **Solution/buffer** | |
|---|---|---|
| Pre-Cultivation | LB-APS broth | |
| | | • 20 g/L BBL Select APS-LB Broth Base |
| Pre-Cultivation | Kanamycin stock | |
| | | • 25 mg/ml Kanamycin |
| Fermentation | Fermentation batch medium | |
| | | • 7.6 g/L (NH₄)₂SO₄, |
| | | • 2.5 g/L KH₂PO₄, |
| | | • 20 g/L Yeast extract, |
| | | • 20 g/L Phytone peptone, |
| | | • 10 µl/L Antifoam 204 |
| Fermentation | Glucose solution (500 g/L) | |
| | | • 550 g/L D (+)-Glucose monohydrate |
| Fermentation | 1000x M2-2 stock | |
| | | • 2.06 g/L CaCl₂ x 2 H₂O |
| | | • 2.78 g/L FeSO₄ x 7 H₂O |
| | | • 203.3 g/L MgCl₂ x 6 H₂O |
| | | • 21 g/L Citric acid x H₂O |
| | | • 0.5 L/L 2000 x Trace metals |
| Fermentation | 2000x Trace metals | |
| | | • 40 mg/L AlCl₃ x 6 H₂O |
| | | • 2.0 mg/L H₃BO₄ |
| | | • 4.0 mg/L KCr(SO₄)₂ x 12 H₂O |
| | | • 20 mg/L CoSO₄ x 7 H₂O |
| | | • 40 mg/L KI |
| | | • 4.0 mg/L CuCl₂ x 2 H₂O |
| | | • 40 mg/L MnSO₄ x H₂O |
| | | • 8.0 mg/L Na₂MoO₄ x 2 H₂O |
| | | • 2.0 mg/L NiSO₄ x 6 H₂O |
| | | • 8.0 mg/L ZnSO₄ x 7 H2O |
| Fermentation | Thiamine stock | |
| | | • 50 g/L Thiamine |
| Fermentation / induction | 1 M IPTG stock | |
| | | • 238.1 g/L IPTG |
| Fermentation / pH control | 2 M NaOH | |
| | | • |
| Fermentation / pH control | 25% H₃PO₄ | |
| | | • |
| Cell wash / Harvesting | 50 mM Tris, pH 7.5 | |
| | | • 6.1 g/L Tris base |
| | | • pH: 7.5 ± 0.1 |

## Claims

1. A fermentation method for producing a recombinant fusion protein comprising or consisting of a first amino acid sequence having at least 90% sequence identity with the amino acid sequence of the N-terminal region of a first group B *Streptococcus* surface protein, said first amino acid sequence being fused to, optionally via linker, a second amino acid sequence having at least 90% sequence identity with the amino acid sequence of the N-terminal region of a second group B *Streptococcus* surface protein,
said method comprising the steps of:
i) growing a host cell, such as an *E. coli* host cell, said host cell comprising a gene encoding said recombinant fusion protein under control of Lacl in a growth medium;
ii) when the cell density reaches an OD₆₀₀ of from about 3.5 to about 50, such as from about 3.5 to about 30, such as from about 3.5 to about 20, such as from about 3.5 to about 10, such as from about 3.5 to about 8, such as from about 4 to about 5, such as about 4.5, adding IPTG to induce expression of said gene encoding said recombinant fusion protein;
iii) harvesting, concentrating and washing the grown cells by tangential flow filtration;
iv) disrupting the cells to prepare a homogenate comprising said recombinant fusion protein.

2. The fermentation method according to claim 1, wherein said IPTG is added to a final concentration of 0.02 mM to about 1 M, such as from about 0.02 mM to about 0.5 M, such as from about 0.02 mM to about 100 mM, such as from about 0.02 mM to about 50 mM, such as from about 0.02 mM to about 10 mM, such as from about 0.02 mM to about 5 mM, such as from about 0.02 mM to about 2 mM ,such as from about 0.03 to about 0.15 mM, such as from about 0.04 to about 0.15 mM, such as from about 0.05 mM to about 0.15 mM.

3. The fermentation method according to claim 1 or 2, wherein said washing of said cells in step ii) is performed by dia-filtration.

4. The fermentation method according to any one of the preceding claims, wherein said cells are disrupted in step iv) by homogenization, such as high pressure homogenization.

5. The fermentation method according to any one of the preceding claims, said method comprising a further step v) of clarifying the homogenate of step iv), such as by tangential flow filtration and dia-filtration, to obtain a protein solution.

6. The fermentation method according to claim 5, said method further comprising a step vi) of filtering the protein solution obtained in step v), such as through a 0.2 µm filter.

7. The fermentation method according to any one of the preceding claims, said method further comprising a pre-cultivation step performed before step i).

8. The fermentation method according to any one of the preceding claims, wherein said *E. coli* host cell is *E. coli* BL21.

9. The fermentation method according to any one of the preceding claims, wherein each of said first and said second group B *Streptococcus* surface protein is selected from the group consisting of Rib protein, Alp1 protein, Alp2 protein, Alp3 protein, Alp4 protein and AlpC protein, with the proviso that said first and second group B *Streptococcus* surface protein are not the same.

10. The fermentation method according to any one of the preceding claims, wherein said recombinant fusion protein is a fusion protein comprising or consisting of the N-terminal regions of the Group B *Streptococcus* Rib and Alpha proteins or the N-terminal regions of the Group B *Streptococcus* Alp1 and Alp2 proteins.

11. The fermentation method according to any one of the preceding claims, wherein said recombinant fusion protein consists of:
i) a first amino acid sequence part consisting of 170 to 178 amino acids, preferably 174 to 175 amino acids, and having at least 90% sequence identity with the amino acid sequence shown in SEQ ID NO: 1;
ii) a second amino acid sequence part consisting of 165 to 174 amino acids, preferably 169 to 170 amino acids, and having at least 90% sequence identity with the amino acid sequence shown in SEQ ID NO: 2; and optionally:
iii) a linker amino acid sequence part consisting of 1 to 20 amino acids and separating the first amino acid sequence part from the second amino acid part,
and wherein the fusion protein preferably consists of 335 to 372 amino acids, preferably 343 to 353 amino acids, more preferably 343 to 347 amino acids.

12. The fermentation method according to any one of the preceding claims, wherein said recombinant fusion protein has an amino acid sequence of SEQ ID NO: 3 or an amino acid sequence having at least 90%, at least 92%, at least 94%, at least 96%, at least 98%, at least 99%, or 100% identity thereto.

13. The fermentation method according to any one of claims 1-10, wherein the recombinant fusion protein consists of
i) a first amino acid sequence part consisting of 168 to 177 amino acids, preferably 172 to 173 amino acids, and having at least 90% sequence identity with the amino acid sequence shown in SEQ ID NO: 4;
ii) a second amino acid sequence part consisting of 168 to 178 amino acids, preferably 172 to 173 amino acids, and having at least 90% sequence identity with the amino acid sequence shown in SEQ ID NO: 5; and optionally
iii) a linker amino acid sequence part consisting of 1 to 20 amino acids and separating the first amino acid sequence part from the second amino acid part, and wherein the fusion protein preferably consists of 335 to 372 amino acids, preferably 343 to 353 amino acids, more preferably 343 to 347 amino acids.

14. The fermentation method according to any one of claims 1-10 and 13, wherein said recombinant fusion protein has an amino acid sequence of SEQ ID NO: 6 or an amino acid sequence having at least 90%, at least 92%, at least 94%, at least 96%, at least 98%, at least 99%, or 100% identity thereto.

15. A recombinant fusion protein as defined in any one of the preceding claims, said recombinant fusion protein being obtained or obtainable by a method according to any one of the preceding claims.
